# EUROPEAN PATENT SPECIFICATION

(11) **EP 4 135 796 B1**
(45) Date of publication and mention of the grant of the patent: **31.12.2025**
(21) Application number: 21720893.3
(22) Date of filing: 31.03.2021
(51) Int. Cl.: A61M 5/32, A61M 5/24, A61M 5/46

(54) **PRE-FILLED SYRINGE SYSTEM AND METHOD**
FERTIGSPRITZENSYSTEM UND VERFAHREN
SYSTÈME DE SERINGUE PRÉREMPLIE ET PROCÉDÉ

(30) Priority: 15.04.2020 US 202063010232 P
(43) Date of publication of application: 22.02.2023
(73) Proprietor: Bayer Healthcare LLC, Whippany, NJ 07981 (US)
(72) Inventor: STOUT, Christopher, Pleasanton, California 94566 (US)
(74) Representative: BIP Patents
(86) International application number: PCT/US2021/025057
(87) International publication number: WO 2021/211295

(56) References cited:
- GB-A- 2 563 027
- US-A- 6 159 184

## Description

### BACKGROUND

The pharmaceutical industry uses various devices and methods for delivering drugs. Pre-filled syringes are a way to quickly and easily deliver a drug directly to the blood stream with maximum efficacy. However, subcutaneous injection using a pre-filled syringe does have its challenges with both devices and methods of injection. For instance, self-injection using a pre-filed syringe requires a user to find and maintain a forty-five-degree angle while inserting the needle through their own skin. In addition, in many instances it also requires the user to maintain a skin fold while they inject themselves. This can be difficult to do correctly and if a wrong angle is obtained the needle could penetrate too deeply. Further, in many pre-filled syringe embodiments there is no possible way for controlling the depth in which the needle is inserted. This can allow the needle to penetrate too deep causing, pain, damage, and exposure to skin or blood complications. Additionally, many pre-filled syringe devices in the art do not provide a needle protection to help eliminate needle sticks after the needle injection is complete. Needle stick injuries post injection are a problem for the pharmaceutical industry.

The pre-filled syringes that exist in the art also have challenges for their uses. For instance, in almost all known devices the user must first insert the needle with their thumb and index finger against the barrel of the syringe while also squeezing the barrel while they insert the needle. Next, to perform the injection, the hand must be repositioned and reoriented while the needle is inside the patient to do the final injection. In some instances, incorrect injections necessitate repeat attempts in order to make the final injection. The device and methods, therefore, provide many challenges to make a correct and effective injection.

Therefore, with these and other challenges in the industry, there is an ongoing need for a pre-filled syringe and method which do not require an injection by the patient at a forty-five-degree angle.

There is still a further need for a pre-filled syringe, system and method which controls the depth of the needle insertion and injection.

Further, there is a need for a pre-filled syringe and system that doesn't require the patient to reposition the needle while the needle is stuck in them.

A pre-filled syringe and system where the syringe provides needle protection after the injection has been completed is known from US 6,159,184. US 6,159,184 discloses a guard for a medical cartridge, such as a unit dose pre-filled glass syringe, comprising a body generally including two elongate rails or similar structures defining a substantially rectangular shape, having a cavity therein adapted to receive a medical cartridge or a pre-filled syringe. It further comprises a protective case or shield which is a tubular member adapted to slidably fit on the body, having open proximal and distal ends. The shield slidably attached to the body are pre-assembled and ready to receive a cartridge therein. The body has a locking mechanism on a proximal end thereof which holds the cartridge therein. The body and shield have cooperating detents and detent pockets which allow the shield to be directed distally, from an unguarded position in which the needle on the cartridge is uncovered for delivery of medication, to a guarded position in which the needle is permanently covered for disposal. However, the system disclosed in US 6,159,184 has certain disadvantages. Therefore, there is a need for a pre-filled syringe and system where the syringe provides needle protection after the injection has been completed, which is easier to assemble and has a simpler design.

These and other problems experienced in the art have been addressed and obviated by the present embodiments.

### SUMMARY

Subject-matter of the present invention is a system for controlled depth of injection in a subject when attached to a prefilled-syringe according to claim 1. Further embodiments are defined in the dependent claims.

### BRIEF DESCRIPTION OF THE DRAWINGS

The skilled artisan will understand that the drawings, described below, are for illustration purposes only. The drawings are not intended to limit the scope of the present teachings or claims in any way.
FIG. 1 - This figure shows a general pre-filled syringe system.
FIG. 2 - This figure shows an exploded view of the pre-filled syringe system and how the components are assembled.
FIG. 3 - This figure shows a side elevation view of the needle shield.
FIG. 4A - This figure shows a view of a first end of the needle shield.
FIG. 4B - This figure shows a view of a second end of the needle shield.
FIG. 5 - This figure shows a side elevation view of the housing.
FIG. 6A - This figure shows the housing and needle shield in a partial assembled releasable locked injection position without the pre-filled syringe.
FIG. 6B - This figure shows the housing and needle shield in a partial assembled and extended shield position without the pre-filled syringe.
FIG. 7A - This figure shows a cross-sectional view of the pre-filled syringe system and needle tip in a locked injection position.
FIG 7B - This figure shows a similar cross-sectional view to 7A, but the pre-filled syringe system and needle tip are in a shield position.
FIG. 8A - This figure shows the locking mechanism of the pre-filled syringe system with seated syringe barrel flange prior to rotation into the locked position.
FIG. 8B - This figure shows the locking mechanism of the pre-filled syringe system with seated syringe barrel flange after it is rotated into the locked position.
FIG. 8C - This figure shows a cross-sectional view of the locking mechanism of the pre-filled syringe system.
FIG 9 - This figure shows the method of injection of the pre-filled syringe system including system preparation 9A, injection of subject 9B, system and needle removal 9C, and system and needle tip placed in shield position 9D.

### DETAILED DESCRIPTION

This disclosure describes embodiments related to a pre-filled syringes and methods of using them.

### Definitions

For the purpose of interpreting this specification, the following definitions will apply. In the event that any definition set forth below conflicts with the usage of that word in any other document, the definition set forth below shall always control for purposes of interpreting this specification and its associated claims unless a contrary meaning is clearly intended (for example in the document where the term is originally used).

Whenever appropriate, terms used in the singular will also include the plural and vice versa. The use of "a" herein means "one or more" unless stated otherwise or where the use of "one or more" is clearly inappropriate.

The use of "or" means "and/or" unless stated otherwise. The use of "comprise," "comprises," "comprising," "include," "includes," and "including" are interchangeable and are not limiting. The terms "such as," "for example," and "e.g." also are not intended to be limiting. For example, the term "including" shall mean "including, but not limited to."

As used herein, the term "about" refers to +/- 10% of the unit value provided.

As used herein, the term "substantially" refers to the qualitative condition of exhibiting a total or approximate degree of a characteristic or property of interest. One of ordinary skill in the biological arts will understand that biological and chemical phenomena rarely, if ever, achieve or avoid an absolute result because of the many variables that affect testing, production, and storage of biological and chemical compositions and materials, and because of the inherent error in the instruments and equipment used in the testing, production, and storage of biological and chemical compositions and materials. The term "substantially" is, therefore, used herein to capture the potential lack of completeness inherent in many biological and chemical phenomena.

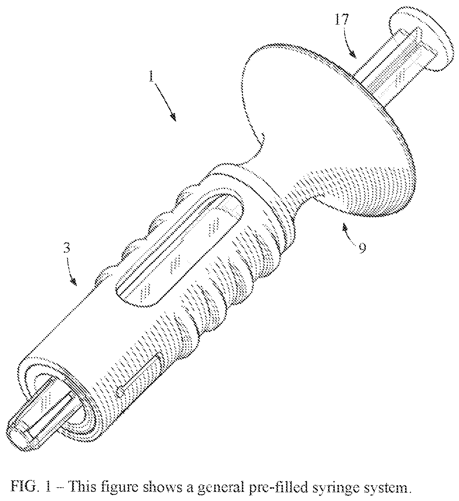

Referring now to the figures, FIG. 1 shows a general perspective view of the pre-filled syringe system 1. The pre-filled syringe system 1, comprises a housing 9, a needle shield 3, and a pre-filled syringe 17. Although the presently described embodiments describe certain components or parts, it is also possible to design the pre-filled syringe system 1 with various other components, orientations, or combinations of elements or parts. Further, each of the components can be further designed to fit in and around each other in various orientations and designs.

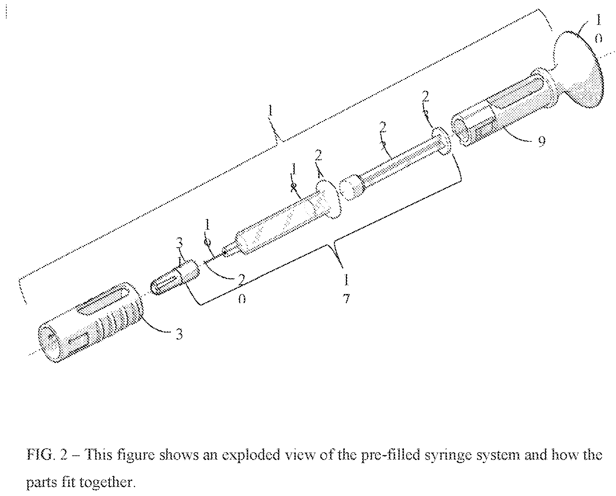

FIG. 2 shows an exploded view of one embodiment of the present invention and how the parts fit together to define the pre-filled syringe system 1. Other embodiments, orientations, and ordering of the parts and assembly can also be possible. As can be seen from FIG. 2 the housing 9 holds the pre-filled syringe 17, and needle shield 3 is then mounted on the housing 9 over the pre-filled syringe 17. The pre-filled syringe 17 can be obtained over the counter or designed for the pre-filled syringe system 1. Various disposable or non-disposable types can be employed with the present embodiments. Pre-filled syringe 17 can comprise a syringe barrel 18 with syringe barrel flange 21, needle 19, needle tip 20, plunger 22 with plunger finger flange 23, and optional needle cap 31. Plunger 22 is designed to fit into syringe barrel 18. Syringe barrel 18 is designed to hold a drug formulation or similar type solution.

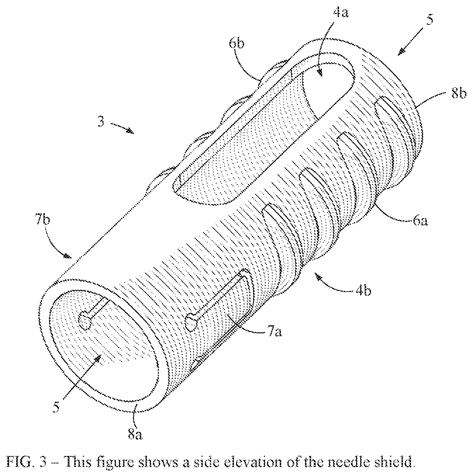

FIG. 3 shows a perspective view of the needle shield 3 of the present embodiments. The needle shield 3 of the present embodiments can be designed to comprise a first needle shield window 4a and a second needle shield window 4b (4b not shown in FIG. 3). The needle shield windows 4a and 4b can be designed so that they are positioned opposite each other. Other orientations and designs are possible. Needle shield 3 can also be designed to comprise a first needle shield finger grip 6a and a second needle shield finger grip 6b as shown in the figures. The first needle shield finger grip 6a and second needle shield finger grip 6b can be designed and positioned opposite each other and orthogonal to the first needle shield window 4a and the second needle shield window 4b. Various types and numbers of finger grips may be employed. Finger grips 6a and 6b are shown in ridge format with two pairs on each side (four on each side or total of 8 ridges). However, other designs are possible. For instance, instead of ridges a series of dimples, notches, or other projection types can be employed for ease of gripping the component.

The needle shield 3 is configured to further comprise a needle shield elongated bore 5 which passes through the entire component. The needle shield elongated bore 5 is designed to receive the housing 9 with or without the inserted pre-filled syringe 17. The needle shield 3 can comprise a single part or various different components that fit together.

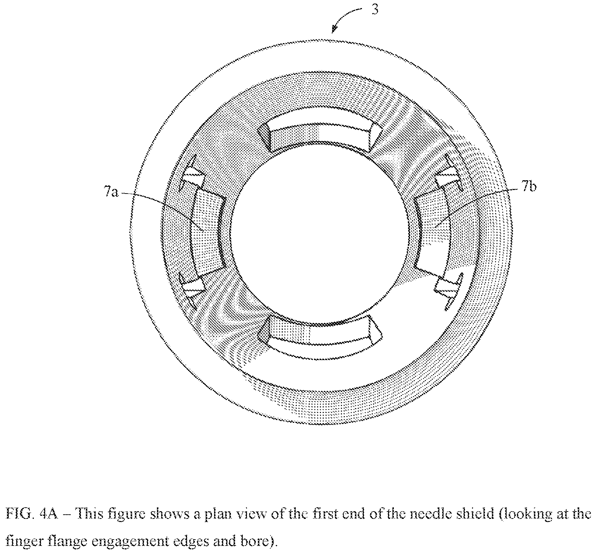

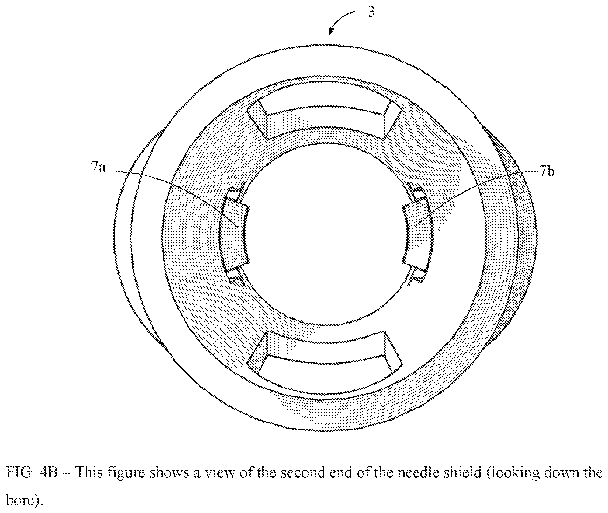

Referring to FIG. 3, the needle shield 3 of the shown embodiment comprises a first locking tab 7a and a second locking tab 7b. The locking tabs 7a and 7b are designed to be inwardly facing. The function of the locking tabs 7a and 7b will be further discussed below in conjunction with how they interact with the complementary locking features of the housing 9. The needle shield 3 can also be designed with various other designs numbers. FIG. 4A shows a plan view of the needle shield 3 from a first end 8a. FIG. 4B shows a plan view of the needle shield 3 from the opposite or second end 8b.

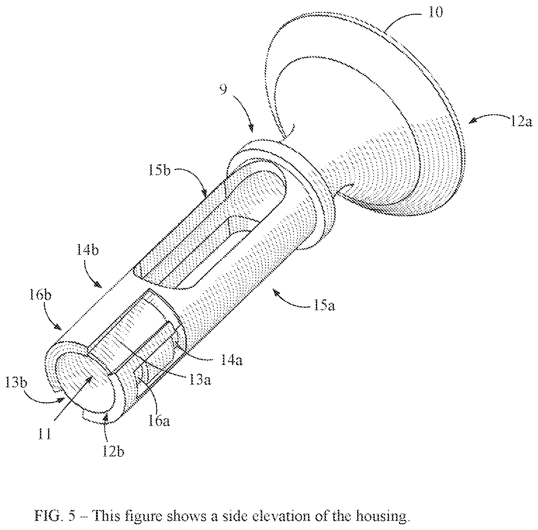

FIG. 5 shows the housing 9 of the present embodiments. The housing 9 can be designed as a single part or a series of components that fit together. The embodiment of housing 9 shown in FIG. 5 comprises a universal finger flange 10. The universal finger flange 10 can be designed in various embodiment sizes and shapes. For purpose of the present embodiments the diameter of the universal finger flange 10 can range from about 5 to about 100 millimeters. The circular and narrow tapering design allows a user to easily grip, hold, and orient the pre-filled syringe system 1 when it is assembled and employed in an injection as will be further discussed below. The universal finger flange 10 as shown in FIG. 5 is designed as part of housing 9. However, it can be imagined in certain embodiments that the universal finger flange 10 can be designed as a separate component with or without a tapered neck and mounted on the housing 9 in various designs and orientations.

Referring again to FIG. 5, the housing 9 comprises a housing elongated bore 11. The housing elongated bore 11 is adapted to receive a pre-filled syringe 17 (See FIG. 2 for further details regarding how the parts fit together) when it is assembled and ready for injection of a pharmaceutical drug. The housing elongated bore 11 runs through the entire housing 9 including the finger flange 10. The housing elongated bore 11 receives the pre-filled syringe 17 via the first end 12a near the finger flange 10.

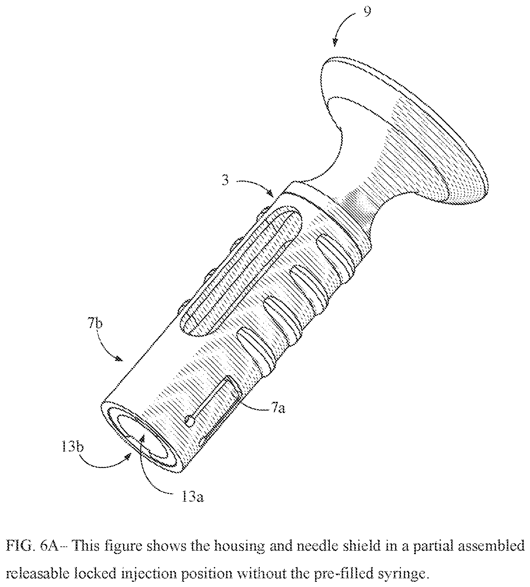

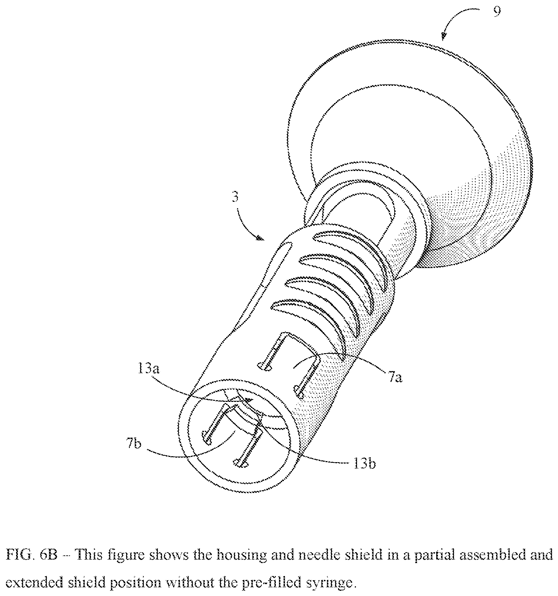

The housing 9 comprises a first ramp 13a and second ramp 13b at the second end 12b. The ramps 13a and 13b are designed to engage the locking tabs 7a and 7b of the needle shield 3 when the components are assembled together. The first ramp 13a and second ramp 13b are designed to taper inwardly toward the central axis of the housing bore 11. Adjacent to the first ramp 13a and second ramp 13b are first locking notch 14a and second locking notch 14b. Locking notches 14a and 14b are designed to receive and engage the locking tabs 7a and 7b of the needle shield 3. In order for the locking tabs 7a and 7b to engage the locking notches 14a and 14b, the needle shield 3 must first be mounted on the housing 9. FIG. 6A shows the housing 9 and the needle shield 3 in a partial assembled releasable locked injection position without the pre-filled syringe 17. It should be noted that both locking notches 14a and 14b are designed to be releasable. This allows the needle shield 3 to then be later adjusted to slide forward and engage locking notches 16a and 16b as will be further discussed below. FIG. 6B shows the housing 9 and the needle shield 3 in a partial assembled shield position without the pre-filled syringe 17. Locking notches 16a and 16b can preferably be designed to be non-releasable. However, it can be imagined in certain embodiments that they can be designed to be releasable if desired.

Adjacent and spaced near the locking notches 14a and 14b are first locking notch 16a and second locking notch 16b. After the needle shield 3 is mounted on the housing 9 it must be rotated into position so that the locking tabs 7a and 7b drop into position and engage locking notches 14a and 14b. This click-feel engagement allows the user to know when the system is engaged and locked. The pre-filled syringe system 1 is then in position to make an injection while maintaining the needle 19 and pre-filled syringe 17 in a defined locked position and orientation. After the injection is complete, the needle shield 3 can then be released and moved away from the housing 9 to only cover a portion of the housing 9 while shielding the needle 19 and engaging first locking notch 16a and second locking notch 16b.

The housing 9 can further comprise a first window housing 15a and second window housing 15b. The first and second window housings 15a and 15b preferably align with the first and second needle window shields 4a and 4b when the pre-filled syringe system 1 is assembled. This alignment is preferable since it allows the subject 30 to view the level and amount of the formulation in the pre-filled syringe 17 at various desired times. For instance, to determine if the pre-filled syringe is filled prior to injection, is injecting the solution or formulation during an injection, or is empty after an injection.

Referring to FIG. 2, the pre-filled syringe 17 comprises a syringe barrel 18 with syringe barrel flange 21, a needle 19 with a needle tip 20 (with optional cap 31), and a plunger 22 having a plunger finger flange 23. The pre-filled syringe 17 can be designed to hold a pharmaceutical product. Pharmaceutical products can comprise both small molecules and biologics in various formulations. For instance, certain biologics which can be used with the present device can comprise biologics for treatment of hemophilia disease such as Factor VII, Factor VIII, Factor IX, Factor X, Factor XI, insulin, Betaseron^{®}, and other similar small molecules, biologics and their combinations which are known and used in the art. Typically syringe barrel 18 can hold from about 0.25 milliliters of formulation to about 2.0 milliliters of formulation. However, it is within the scope of the present embodiments that various volumes can be employed.

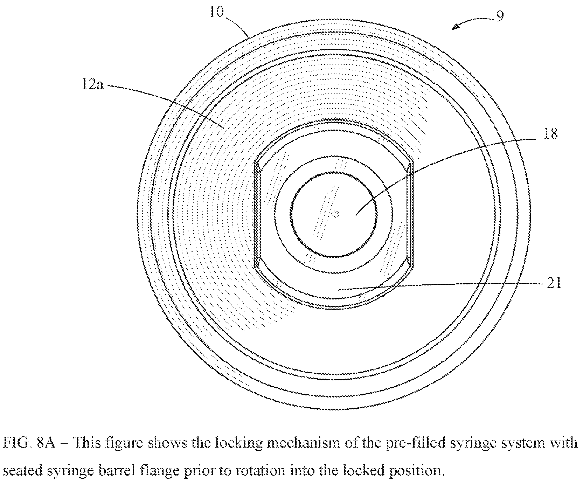

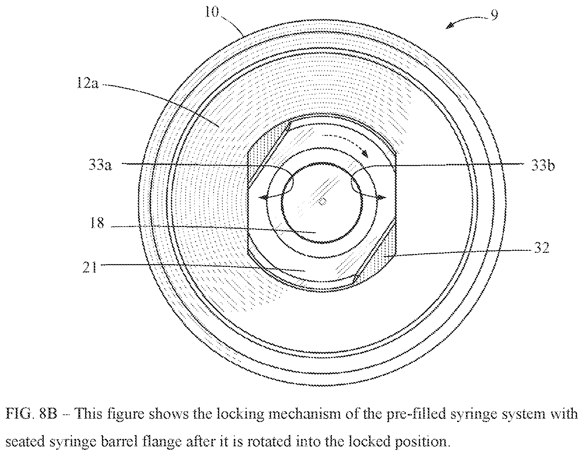

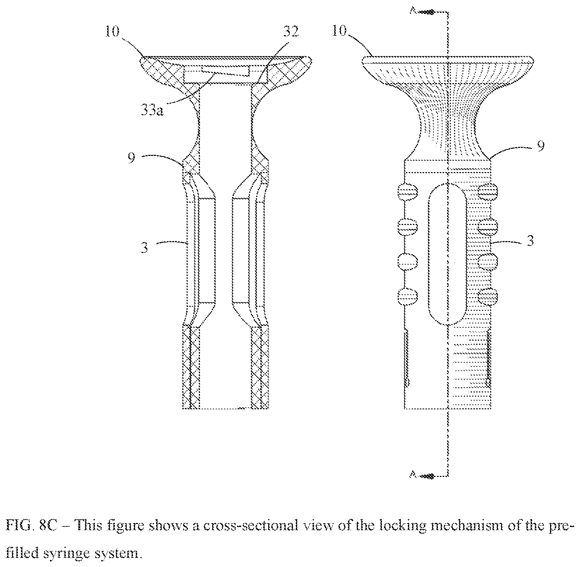

The pre-filled syringe system 1, can further comprise a locking mechanism 33 for attaching the pre-filled syringe 17 to the housing 9 and pre-filled syringe system 1. The locking mechanism 33 can comprise syringe barrel flange 21, mounting surface 32, first locking surface 32a, and second locking surface 32b (See FIG. 8 for details and assembly). It can be imagined that locking mechanism 33 can comprise various parts, organizations, and designs for attaching the pre-filled syringe 17 to the housing 9 and pre-filled syringe system 1. Further, it could be imagined that the locking mechanism 33 could comprise a portion of the pre-filled syringe system 1 (as shown in the present embodiments) or comprise one or more separate parts for attaching the pre-filled syringe 17 onto the housing 9 and pre-filled syringe system 1. Further, barrel flange 21 could be designed in different shapes or sizes for mounting and seating on the first and second locking surfaces 32a and 32b respectively. The first locking surface 32a and the second locking surface 32b could also be designed as a tab or similar type structure.

The assembly of the embodiments can begin by placing a pharmaceutical formulation into the syringe barrel 18 of the pre-filled syringe 17. The plunger 22 then is inserted into the syringe barrel 18 behind the pharmaceutical formulation so it contacts or nearly contacts the formulation and any air bubbles are removed from the needle tip 20. Optional cap 31 can be included in the assembly and remain mounted on needle tip 19 to prevent accidental needle sticks. However, optional cap 31 must be removed prior to the injection of the subject 30.

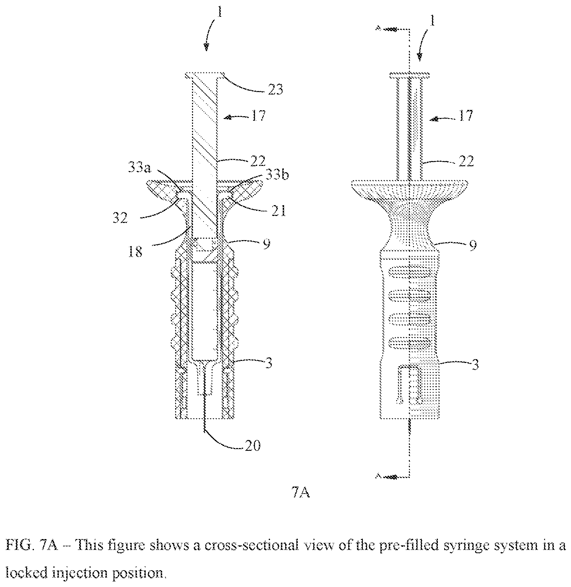

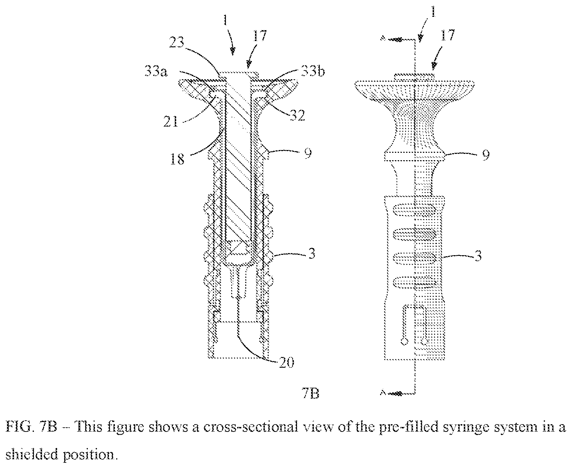

The plunger 22 then remains in the loaded, pre-injection position. The pre-filled syringe 17 with formulation is then inserted into the housing bore 11 via the end with the universal finger flange 10 (first end 12a). The pre-filled syringe 17 slides all the way into the housing bore 11 until the syringe barrel 18 and syringe barrel flange 21 are seated against mounting surface 32 of the universal finger flange 10 (See FIG. 7 and 8). The mounting surface 32 can be designed to be grooved, indented, tapered, dimpled or in other similar type designs or shapes to engage syringe barrel 18 and syringe barrel flange 21 of the pre-filled syringe 17.

The pre-filled syringe 17 should preferably fit tight enough via friction that it does not disengage or fall out of the housing 9. In certain embodiments (as show in the FIGS. 7 and 8) locking mechanism 33 may be employed for attaching the pre-filled syringe 17 to the housing 9 and pre-filled syringe system 1. For instance, after the pre-filled syringe 17 is assembled, mounted in housing 9, and pre-filled syringe flange 21 is seated on mountain surface 32 of the universal finger flange, the locking mechanism 33 can be locked. For instance, the syringe barrel flange 21 can be rotated to engage the first locking surface 32a and the second locking surface 32b to securely lock and attach the pre-filled syringe 17 to the housing 9 and pre-filled syringe system 1 (See FIG. 8B and 8C). It can be imagined that locking mechanism 33 can be designed in various releasable and non-releasable locking designs and formats.

The plunger 22 of the pre-filled syringe 17 remains in a loaded pre-injection position during this process. Next, the needle shield 3 is mounted on the housing 9 via sliding it over the housing using the needle shield bore 5. The first and second locking tabs 7a and 7b of the needle shield 3 slide along first and second ramps 13a and 13b until they contact the housing 9. The mounting orientation of the needle shield 3 is such that the end having the first and second finger grips 6a and 6b is mounted first on the housing 9. Once the needle shield 3 has been mounted on the housing 9 it is then rotated so that the first and second tabs 7a and 7b drop into the first and second locking notches 14a and 14b. In this orientation, the first and second needle shield windows 4a and 4b are aligned with the first and second housing windows 15a and 15b so that the syringe barrel 18 of the pre-filled syringe 17 with formulation can be viewed. The pre-filled syringe system 1 is now in locked injection mode and ready for use. The user at this point simply needs to remove the needle cap 31 from the pre-filled syringe needle tip 20 and perform the injection. FIG. 7A and 7B show cross-sectional views of the pre-filled syringe system 1 and pre-filled syringe 17 in the locked and shielded positions and how the needle tip 20 can extend at a defined length from the needle shield 3 and pre-filled syringe system 1 (See FIG. 7A) or be shielded by needle shield 3 (See FIG. 7B).

Having now described the components and their assembly, a description of the method of using the pre-filled syringe system 1 is now in order.

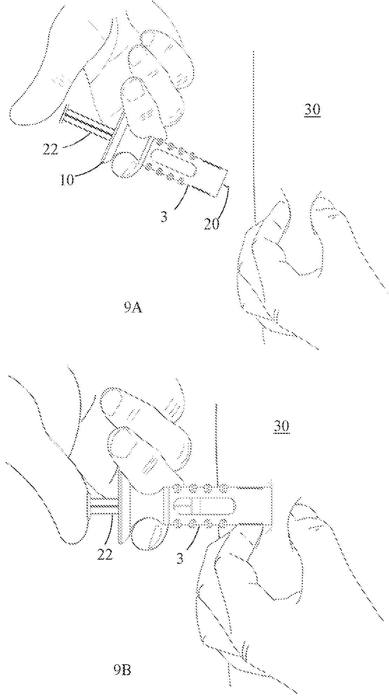

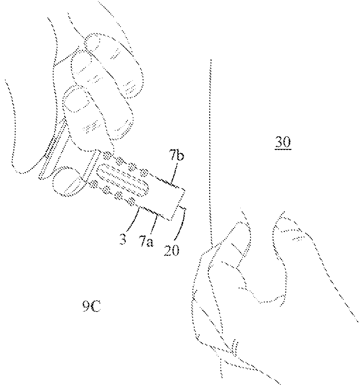

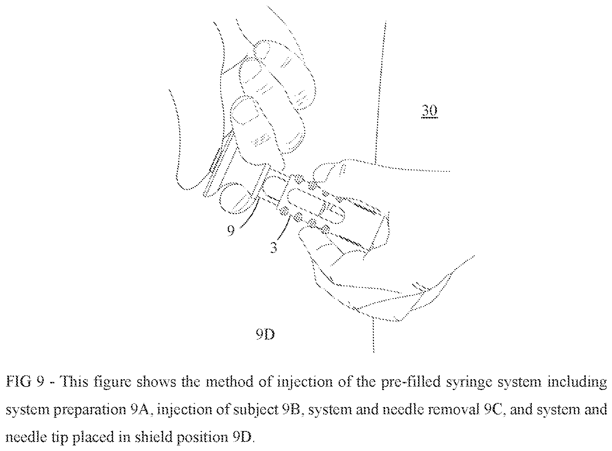

FIG. 9 shows the method of injection of the present embodiments. As previously discussed, the pre-filled syringe system 1 is assembled with a pre-filled syringe 17 having a pharmaceutical formulation. The pre-filled syringe plunger 22 is also in an extended and loaded position once the drug has been loaded. Once the needle shield 3 has been positioned on the housing 9 and placed into the locked position with extended needle tip 20, the device is ready for use. The extent of protrusion of the needle tip 20 can be dependent upon the length of the needle 19, and the size of the needle shield 3. The extension length can be anywhere from 2 to 30 millimeters (as measured from the edge of the needle shield 3) or preferably from about 4 millimeters to about 8 millimeters, depending up the desired depth of injection into the subject.

The subject takes the entire pre-filled syringe system 1 in one hand with fingers around the universal finger flange 10, (FIG 9A). The needle tip 20 and a portion of the needle 19 is then inserted into the subject 30. Once the needle 19 is inserted it continues until the needle shield 3 contacts the subject 30 and can't penetrate any deeper into the subject's skin (FIG. 9B). Next, the plunger 22 of the pre-filled syringe 17 is depressed and the formulation contents is injected into the subject 30. In the present context, the pharmaceutical drug is delivered into the subject 30 at the desired position and depth. After the injection has been completed the pre-filled syringe system 1 and pre-filled syringe 17 are then removed from the subject 30 (See FIG. 9C). At this point the needle 19 and needle tip 20 are still extended from the needle shield 3 in locked position (as previously described with releasable first locking tab 7a and second locking tab 7b positioned in first locking notch 14a and second locking notch 14b). Pressure is then applied to the sides of needle shield 3 to release first locking tab 7a and second locking tab 7b from the first locking notch 14a and the second locking notch 14b so that needle shield 3 can be slid forward on housing 9 so the first locking tab 7a and second locking tab 7b of the needle shield 3 engage the first locking notch 16a and second locking notch 16b of the housing 9 (See FIG. 9D). As previously discussed, the first and second locking notches 14a and 14b are preferably designed for releasable movement of the needle shield 3 while the first and second locking notches 16a and 16b are not. In this second position, the needle shield 3 now completely shields and encloses the needle 19 and needle tip 20 so they no longer extend outside the pre-filled syringe system 1 to possibly injure the subject 30. The pre-filled syringe system 1 and pre-filled syringe 17 can then be safely disposed of without risk of injuring the subject 30 or others.

## Claims

1. A system for controlled depth of injection in a subject (30) when attached to a prefilled-syringe (17) comprising:
(a) a housing (9) for holding the pre-filled syringe (17), the housing (9) having a first end (12a) near a finger flange (10) and a second end (12b); and
(b) a needle shield (3) mounted on the housing (9) for releasable movement from a locked injection position to a shield position,
wherein
the needle shield (3) has a first inwardly facing locking tab (7a) and a second inwardly facing locking tab (7b);
the housing (9) comprises at its outer surface:
(i) a first ramp (13a) and second ramp (13b) adapted to engage the first locking tab (7a) and the second locking tab (7b) for mounting the needle shield (3) on the housing (9);
(ii) adjacent and angular offset to the first ramp (13a) and the second ramp (13b) a first locking notch (14a) and a second locking notch (14b) designed to receive and releasably engage the first locking tab (7a) and second locking tab (7b) when the needle shield (3) has been mounted and rotated into the injection position;
(iii) a first additional locking notch (16a) and a second additional locking notch (16b) designed to engage the needle shield (3) when the needle shield (3) is moved into the shield position,
wherein the first additional locking notch (16a) and the second additional locking notch (16b) are adjacent and spaced in direction to the second end (12b) from and aligned with the first locking notch (14a) and the second locking notch (14b).

2. A system as recited in claim 1, wherein the first additional locking notch (16a) and the second additional locking notch (16b) are designed to non-releasably receive and engage the first locking tab (7a) and the second locking tab (7b).

3. A system as recited in claim 1 or 2, wherein the first locking tab (7a) and the second locking tab (7b) are on opposite sides of the needle shield (3) and the first locking notch (14a) and the second locking notch (14b) are on opposite sides of the housing (9).

4. A system as recited in one of the claims 1 to 3, wherein the housing (9) further comprises a universal finger flange (10), wherein preferably the universal finger flange (10) is circular and has a tapered neck.

5. A system as recited in one of the claims 1 to 4, wherein the housing (9) comprises one single part and/or the needle shield (3) comprises one single part.

6. A system as recited in one of the claims 1 to 5, wherein the needle shield (3) comprises one or more finger grips (6a, 6b).

7. A system as recited in one of the claims 1 to 6 further comprising:
a pre-filled syringe (17) having a needle tip (20) and a plunger (22) for injecting the contents of the pre-filled syringe (17).

8. A system as recited in claim 7, wherein the needle tip (20) extends from the needle shield (3) and pre-filled syringe system (1) from about 4 millimeters to about 8 millimeters.

9. A system as recited in claim 7 or 8, wherein the housing (9) comprises at least one window (15a, 15b) for viewing the contents of the pre-filled syringe (17).

10. A system as recited in one of the claims 7 to 9, wherein the needle shield (3) comprises at least one needle shield window (4a, 4b) for viewing the injection contents in the pre-filled syringe (17).

11. A system as recited in one of the claims 7 to 10, further comprising a locking mechanism (33) for attaching the pre-filled syringe (17) to the housing (9) and the pre-filled syringe system (1).

## Patentansprüche

1. System für kontrollierte Tiefe der Injektion in ein Subjekt (30), wenn an einer vorgefüllten Spritze (17) angebracht, umfassend:
(a) ein Gehäuse (9) zum Halten der vorgefüllten Spritze (17), wobei das Gehäuse (9) ein erstes Ende (12a) in der Nähe eines Fingerflansches (10) und ein zweites Ende (12b) hat; und
(b) einen Nadelschutz (3), der zur lösbaren Bewegung aus einer verriegelten Injektionsposition in eine Schutzposition an dem Gehäuse (9) montiert ist,
wobei der Nadelschutz (3) eine erste nach innen weisende Verriegelungszunge (7a) und eine zweite nach innen weisende Verriegelungszunge (7b) hat;
das Gehäuse (9) an seiner Außenseite umfasst:
(i) eine erste Rampe (13a) und eine zweite Rampe (13b), die dazu angepasst sind, die erste Verriegelungszunge (7a) und die zweite Verriegelungszunge (7b) zum Befestigen des Nadelschutzes (3) an dem Gehäuse (9) in Eingriff zu nehmen;
(ii) neben und winklig versetzt von der ersten Rampe (13a) und der zweiten Rampe (13b) eine erste Verriegelungskerbe (14a) und eine zweite Verriegelungskerbe (14b), die dazu ausgestaltet sind, die erste Verriegelungszunge (7a) und die zweite Verriegelungszunge (7b) aufzunehmen und lösbar in Eingriff zu nehmen, wenn der Nadelschutz (3) montiert und in die Injektionsposition gedreht wurde;
(iii) eine erste zusätzliche Verriegelungskerbe (16a) und eine zweite zusätzliche Verriegelungskerbe (16b), die dazu ausgestaltet sind, den Nadelschutz (3) in Eingriff zu nehmen, wenn der Nadelschutz (3) in die Schutzposition bewegt wird,
wobei die erste zusätzliche Verriegelungskerbe (16a) und die zweite zusätzliche Verriegelungskerbe (16b) benachbart und in Richtung des zweiten Endes (12b) von der ersten Verriegelungskerbe (14a) und der zweiten Verriegelungskerbe (14b) beabstandet und darauf ausgerichtet sind.

2. System nach Anspruch 1, wobei die erste zusätzliche Verriegelungskerbe (16a) und die zweite zusätzliche Verriegelungskerbe (16b) dazu ausgestaltet sind, die erste Verriegelungszunge (7a) und die zweite Verriegelungszunge (7b) nicht-lösbar aufzunehmen und in Eingriff zu nehmen.

3. System nach Anspruch 1 oder 2, wobei die erste Verriegelungszunge (7a) und die zweite Verriegelungszunge (7b) auf gegenüberliegenden Seiten des Nadelschutzes (3) liegen und die erste Verriegelungskerbe (14a) und die zweite Verriegelungskerbe (14b) auf gegenüberliegenden Seiten des Gehäuses (9) liegen.

4. System nach einem der Ansprüche 1 bis 3, wobei das Gehäuse (9) ferner einen universellen Fingerflansch (10) umfasst, wobei der universelle Fingerflansch (10) vorzugsweise kreisförmig ist und einen konischen Hals hat.

5. System nach einem der Ansprüche 1 bis 4, wobei das Gehäuse (9) aus einem einzigen Teil besteht und/oder der Nadelschutz (3) aus einem einzigen Teil besteht.

6. System nach einem der Ansprüche 1 bis 5, wobei der Nadelschutz (3) einen oder mehrere Fingergriffe (6a, 6b) umfasst.

7. System nach einem der Ansprüche 1 bis 6, ferner umfassend:
eine vorgefüllte Spritze (17) mit einer Nadelspitze (20) und einem Kolben (22) zum Injizieren des Inhalts der vorgefüllten Spritze (17).

8. System nach Anspruch 7, wobei die Nadelspitze (20) etwa 4 Millimeter bis etwa 8 Millimeter aus dem Nadelschutz (3) und dem vorgefüllten Spritzensystem (1) herausragt.

9. System nach Anspruch 7 oder 8, wobei das Gehäuse (9) mindestens ein Fenster (15a, 15b) zum Betrachten des Inhalts der vorgefüllten Spritze (17) umfasst.

10. System nach einem der Ansprüche 7 bis 9, wobei der Nadelschutz (3) mindestens ein Nadelschutzfenster (4a, 4b) zum Betrachten des Injektionsinhalts in der vorgefüllten Spritze (17) umfasst.

11. System nach einem der Ansprüche 7 bis 10, ferner umfassend einen Verriegelungsmechanismus (33) zum Anbringen der vorgefüllten Spritze (17) an dem Gehäuse (9) und dem vorgefüllten Spritzensystem (1).

## Revendications

1. Système pour une profondeur d'injection contrôlée chez un sujet (30) lorsqu'il est fixé à une seringue préremplie (17) comprenant :
(a) un boîtier (9) pour maintenir la seringue préremplie (17), le boîtier (9) ayant une première extrémité (12a) proche d'une bride de doigt (10) et une seconde extrémité (12b) ; et
(b) une protection d'aiguille (3) montée sur le boîtier 9) pour un mouvement libérable d'une position d'injection verrouillée vers une position de protection, la protection d'aiguille (3) ayant une première patte de verrouillage (7a) tournée vers l'intérieur et une seconde patte de verrouillage (7b) tournée vers l'intérieur ;
le boîtier (9) comprenant au niveau de sa surface extérieure :
(i) une première rampe (13a) et une seconde rampe (13b) adaptées pour venir en prise avec la première patte de verrouillage (7a) et la seconde patte de verrouillage (7b) pour le montage de la protection d'aiguille (3) sur le boîtier (9) ;
(ii) de manière adjacente et décalée angulairement de la première rampe (13a) et de la seconde rampe (13b), une première encoche de verrouillage (14a) et une seconde encoche de verrouillage (14b) conçues pour recevoir et venir en prise de manière amovible avec les première patte de verrouillage (7a) et seconde patte de verrouillage (7b) lorsque la protection d'aiguille (3) a été montée et tournée dans la position d'injection ;
(iii) une première encoche de verrouillage supplémentaire (16a) et une seconde encoche de verrouillage supplémentaire (16b) conçues pour venir en prise avec la protection d'aiguille (3) lorsque la protection d'aiguille (3) est déplacée en position de protection,
la première encoche de verrouillage supplémentaire (16a) et la seconde encoche de verrouillage supplémentaire (16b) étant adjacentes et espacées en direction de la seconde extrémité (12b) et alignées avec la première encoche de verrouillage (14a) et la seconde encoche de verrouillage (14b).

2. Système selon la revendication 1, la première encoche de verrouillage supplémentaire (16a) et la seconde encoche de verrouillage supplémentaire (16b) étant conçues pour recevoir et venir en prise de manière non libérable avec la première patte de verrouillage (7a) et la seconde patte de verrouillage (7b).

3. Système selon la revendication 1 ou 2, la première patte de verrouillage (7a) et la seconde patte de verrouillage (7b) étant sur des côtés opposés de la protection d'aiguille (3) et la première encoche de verrouillage (14a) et la seconde encoche de verrouillage (14b) étant sur des côtés opposés du boîtier (9).

4. Système selon l'une quelconque des revendications 1 à 3, le boîtier (9) comprenant en outre une bride de doigt universelle (10), la bride de doigt universelle (10) étant de préférence circulaire et ayant un col conique.

5. Système selon l'une quelconque des revendications 1 à 4, le boîtier (9) comprenant une seule pièce et/ou la protection d'aiguille (3) comprenant une seule pièce.

6. Système selon l'une des revendications 1 à 5, la protection d'aiguille (3) comprenant une ou plusieurs poignées de doigts (6a, 6b).

7. Système selon l'une des revendications 1 à 6, comprenant en outre :
une seringue préremplie (17) comprenant une pointe d'aiguille (20) et un piston (22) permettant d'injecter le contenu de la seringue préremplie (17).

8. Système selon la revendication 7, la pointe d'aiguille (20) s'étendant depuis la protection d'aiguille (3) et le système de seringue préremplie (1) d'environ 4 millimètres à environ 8 millimètres.

9. Système selon la revendication 7 ou 8, le boîtier (9) comprenant au moins une fenêtre (15a, 15b) pour visualiser le contenu de la seringue préremplie (17).

10. Système selon l'une des revendications 7 à 9, la protection d'aiguille (3) comprenant au moins une fenêtre de protection d'aiguille (4a, 4b) pour visualiser le contenu injecté dans la seringue préremplie (17).

11. Système selon l'une des revendications 7 à 10, comprenant en outre un mécanisme de verrouillage (33) pour fixer la seringue préremplie (17) au boîtier (9) et au système de seringue préremplie (1).
